# EUROPEAN PATENT SPECIFICATION

(11) **EP 0 611 304 B1**
(45) Date of publication and mention of the grant of the patent: **15.09.1999**
(21) Application number: 92900586.6
(22) Date of filing: 05.11.1991
(51) Int. Cl.: A61K 31/715

(54) **USE OF ACETYLATED MANNAN (ACEMANNAN) FOR THE REGULATION OF BLOOD CHOLESTEROL LEVELS AND FOR REMOVING PLAQUES IN BLOOD VESSELS**
VERWENDUNG VON ACETYLIERTEM MANNAN (ACEMANNAN) ZUR REGULIERUNG VON BLUTCHOLESTERIN-SPIEGELN UND ENTFERNUNG VON PLAQUES IN BLUTGEFÄSSEN
UTILISATION DE MANNANE ACETYLE (ACEMANNAN) DANS LA REGULATION DES TAUX DE CHOLESTEROL DANS LE SERUM ET POUR L'ELIMINATION DE PLAQUES DES VAISSEAUX SANGUINS

(43) Date of publication of application: 24.08.1994
(60) Divisional application: 99200519.9
(73) Proprietor: CARRINGTON LABORATORIES, INC., Irving, TX 75038 (US)
(72) Inventor: McANALLEY, Bill H., Grand Prairie, TX 75052 (US); CARPENTER, Robert H., Bastrop, TX 78602 (US); McDANIEL, Harley R., Dallas, TX 75205 (US)
(74) Representative: Howick, Nicholas Keith
(86) International application number: US9108204
(87) International publication number: WO9308810

(56) References cited:
- WO-A-87/00052
- WO-A-90/01253
- Int. Conf. AIDS, vol. 6, no. 2, 1990, M.C. KEMP et al.: "Concentration-dependent inhibition of AIDS virus replication and pathogenesis by acemannan in vitro", page 315, abstract no. 1007, see abstract
- Dissertation Abstracts International, vol. 52, no. 2, August 1991, A.D. CHINNAH et al.: "Evaluation of the antiviral, adjuvant and immunomodulatory effects of a beta-(1,4)-linked polymannose (acemannan)", page 694-B, see abstract
- International Journal of Immunopharmacology, vol. 10, no. 8, 1988, D. WOMBLE et al.: "Enhancement of allo-responsiveness of human lymphocytes by acemannan (Carrisyn TM)", pages 967-974, see abstract; pages 967-968; pages 972-973: "Discussion"
- Fed. Am. Soc. Exp. Biol. J., vol. 2, no. 4, 1988, J. MEASEL et al.: "The effect of Carrisyn on the immune system", abstract no. 2239, see abstract
- Antiviral Res. O., suppl. 1, 1990, M.C. KEMP et al.: "In vitro evaluation of the antiviral effects of acemannan on the replication and pathogenesis of HIV-1 and other enveloped viruses: modification of the processing of glycoprotein precursors", page 83, abstract no. 84, see abstract
- Antiviral Res. O., suppl. 1, 1990, H.R. McDANIEL et al.: "Extended survival and prognostic criteria for acemannan (ACE-M) treated HIV-1 patiens(pts)", page 117, abstract no. 147, see abstract
- American Journal of Clinical Pathology, vol. 88, no. 4, October 1987, H.Reg. McDANIEL et al.: "A clinical pilot study using Carrisyn TM in the treatment of acquired immunodeficiency syndrome (AIDS)", page 534, see abstract
- Drugs of Today, vol. 25, no. 1, January 1989, Y.W. CHIEN et al.: "AIDS and chemotherapy", pages 19-25, see page 23, paragraph 2
- Mol. Biother., vol. 3, no. 1, March 1991, M.A. SHEETS et al.: "Studies of the effect of acemannan on retrovirus infections: clinical stabilization of feline leukemia virus-infected cats", pages 41-45, see abstract; pages 41-42,44-55
- Mol. Biother., vol. 3, no. 2, June 1991, S.Y. PENG et al.: "Decreased mortality of norman murine sarcoma in mice treated with the immunomodulator, acemannan TM", pages 79-87, see abstract; pages 79-80,85-86

## Description

### BACKGROUND OF THE INVENTION

### A. Field of the Invention

The invention pertains to the use of acetylated mannans for the manufacture of a medicament to regulate blood cholesterol levels or to remove plaques formed in blood vessels of an animal.

### B. Description of the General Background Information

Aloe is a member of the lily family. Aloe vera contains two major liquid sources, a yellow latex (exudate) and the clear gel (mucilage). The dried exudate of Aloe barbadensis Miller leaves is referred to as aloe. The commercial name is Curacao aloe. It is composed mainly of aloin, aloe-emodin and phenols. Bruce, South African Medical Journal, 41:984 (1967); Morrow et al., Archives of Dermatology, 116:1064-1065 (1980); Mapp et al., Planta Medica, 18:361-365 (1970); Rauwald, Archives Pharmazie, 315:477-478 (1982). A number of phenolics, including anthraquinones and their glycosides, are known to be pharmaceutically active. Bruce, Excelsa, 5:57-68 (1975); Suga et al., Cosmetics and Toiletries, 98:105-108 (1983).

The mucilaginous jelly from the parenchymal cells of the plant is referred to as Aloe vera gel. There are generally no anthraquinones to decompose and cause discoloration of the gel unless the gel is contaminated by an improper processing technique. Aloe vera gel is about 98.5% water by weight. More than 60% of the total solid is made up of polysaccharides of carbohydrate origin. Organic acids and inorganic compounds, especially calcium oxalate, account for the remainder of the solid.

Aloe vera was the traditional medicine of many cultures as an anthelmintic, cathartic and stomachic and was used inter alia for leprosy, burns and allergic conditions. Cole et al., Archives of Dermatology and Syphilology, 47:250 (1943); Chopra et al., Glossary of Indian Medicinal Plants, Council of Scientific and Industrial Research, New Delhi (1956); Ship, Journal of the American Medical Association, 238(16):1770-1772 (1977); Morton, Atlas of Medicinal Plants of Middle American Bahamas to Yucatan, Charles C. Thomas Publisher, 78-80 (1981); Diez-Martinez, La Zabila, Communicado NO. 46 Sobre Recursos Bioticos Potenciales del Pais, INIREB, Mexico (1981); Dastur, Medicinal Plants of India and Pakistan: D.B. Taraporevala Sons & Co., Private Ltd., Bombay 16-17 (1962).

Aloe vera has been featured extensively in the field of dermatology, especially for treating radiation-caused skin conditions. Mackee, X-rays and Radium in the Treatment of Diseases of the Skin, 3rd Ed., Lea and Febiger, Philadelphia, 319-320 (1938); Rovatti et al., Industrial Medicine and Surgery, 28:364-368 (1959); Zawahry et al., Quotations From Medical Journals on Aloe Research, Ed. Max B. Skousen, Aloe Vera Research Institute, Cypress, California, 18-23 (1977); Cera et al., Journal of the American Animal Hospital Association, 18:633-638 (1982). The body of scientific literature documenting medical applications in digestive problems, as a virucidal, bactericidal and fungicidal agent and in gynecological conditions is extensive and has been adequately reviewed by Grindley et al., [Journal of Ethnopharmacology, 16:117-151 (1986)].

Depending on the way the leaves are processed, mucilage and sugars are the major components of the dehydrated gel. The sugars found are galactose, glucose, mannose, rhamnose, xylose and uronic acids. Although reports conflict, the mucilage is mainly composed of mannan or glucomannan. Eberendu et al., The Chemical Characterization of Carrisyn^{®} (in preparation); Mandal et al., Carbohydrate Research, 86:247-257 (1980b); Roboz et al., Journal of the American Chemical Society, 70:3248-3249 (1948); Gowda et al., Carbohydrate Research, 72:201-205 (1979); Segal et al., Lloydia, 31:423 (1968).

Prior to this work, the controversy over the identity of the active substance(s) in Aloe vera had not been settled. It is therefore important to clearly distinguish between the components present in the gel and those found in the exudates. A majority of the gel is a mucilage of mainly polysaccharide nature with minor amounts of various other compounds. It has been observed that in some of the activities there may be some synergistic action between the polysaccharide base and other components. Leung, Excelsa 8:65-68 (1978); Henry, Cosmetics and Toiletries, 94:42-43, 46, 48, 50 (1979). For example, several workers report that the effective components for wound healing may be tannic acid [Freytag, Pharmazie, 9:705 (1954)] and a kind of polysaccharide. Kameyama, Wound-healing compositions from Aloe arborescens extracts. Japanese Patent#7856995, (1979).

However, there are many examples in the literature indicating that polysaccharides can exhibit pharmacological and physiological activities without help from other components. Gialdroni-Grassi, International Archives of Allergy and Applied Immunology, 76(Suppl. 1):119-127 (1985); Ohno et al., Chemical and Pharmaceutical Bulletin, 33(6):2564-2568 (1985); Leibovici et al., Chemico-Biological Interactions, 60:191-200 (1986); Ukai et al., Chemical and Pharmaceutical Bulletin, 31:741-744 (1983); Leibovici et al., Anticancer Research, 5:553-558 (1985). One such example relates to development of atherosclerosis. Hyperlipidemia in the general population and especially in familial hypercholesterolemia is associated with coronary heart disease and death. In countries where dietary fiber intake is high, atherosclerosis appears to be uncommon. Trowell et al., Editors, Refined Carbohydrate Foods and Disease, London, Academic Press, 207 (1975). Pectin and guar are reported to lower cholesterol in normal and hyperlipidemic patients. Kay et al., American Journal of Clinical Nutrition, 30:171-175 (1977). Locust bean gum, a polysaccharide composed of mannose and galactose, decreased the plasma lipoprotein cholesterol concentrations in both normal and familial hypercholesterolemic subjects. Zavoral et al., American Journal of Clinical Nutrition, 38:285-294 (1983). Addition of guar gum to carbohydrate meals decreased the postprandial rise of glucose in both normal and diabetic subjects. Jenkins et al., Lancet, 2:779-780 (1977). Kuhl et al., in Diabetes Care, 6(2):152-154 (1983) demonstrated that guar gum exhibited glycemic control of pregnant insulin-dependent diabetic patients.

The antitumor activity of polysaccharides has been widely reported. Polysaccharides prepared from Lentinus cyathiformis are known to increase host defense against tumors. Rethy et al., Annales Immunologiae Hungaricae, 21:285-290 (1981). There are several reports that polysaccharides from mushroom, yeast or bacterial extracts can elicit a high degree of host defense activity against viral and tumor infestations. Chihara, Nature, 222:687 (1969); Shwartzman et al., Proceedings of the Society for Experimental Biology and Medicine, 29:737-741 (1932); Suzuki et al., Journal of Pharmacobio-Dynamics, 7(7):492-500 (1984), also reported antitumor activity of a polysaccharide fraction (GF-1) extracted from cultured fruiting bodies of a fungus, Grifola frondosa. This fraction showed equivalent, high levels of inhibiting activity when administered intraperitoneally (IP), intravenously (IV) and intratumorally (IT). However, oral administration (PO) was not effective. The GF-1 fraction also exhibited antitumor action against the solid form of Meth A fibrosarcoma and MM 46 carcinoma in mice. Lentinan, which is a 6-branched β-1-3-linked glucan similar to GF-1, was ineffective against Meth A fibrosarcoma. Chihara, "The antitumor polysaccharide Lentinan: an overview;" Manipulation of Host Defense Mechanisms; Ed. by Aoki et al., Excerpta Medica, North Holland, 1-16 (1981); Sasaki et al., Carbohydrate Research,47(1):99-104 (1976). Synthesized branched polysaccharides were reported to demonstrate activities against tumors. Matsuzaki et al., Makromol. Chem., 186(3):449-456 (1985). Matsuzaki et al. [Makromol. Chem., 187(2):325-331 (1986)] synthesized branched polysaccharides, which showed significant activities, from ivory nut mannan (β-(1-4)-D-mannopyranose) and β-(1-4)-linked glucomannan. A partially acetylated linear β-(1-3)-D-mannan extracted from fruit bodies of Dictyophoria indusiata Fisch, also exhibited antitumor activity. Hara, Carbohydrate Research, 143:111 (1982). It appears that antitumor action depends on the type of polymer main chain and its degree of polymerization, because β-(1-3)-glucan-type polymers show higher antitumor activity than β-(1-4)-glucan and hemicellulosic polymers. Matsuzaki et al., Makromol. Chem., 187:325-331 (1986). A carboxymethylated derivative of β-(1-3)-glucan obtained from bacterial culture filtrate caused severe cell loss from established sarcoma 180 tumors within 2 hours after the injection of the derivative. Baba, Journal of Immunopharmacology, 8(6):569-572 (1986). The same author observed a compensatory increase in polymorphonuclear leukocytes due to injection of the substance. Incidentally, bestatin, a dipeptide known to possess immune-modulating and antitumor activity [Ishizuka, Journal of Antibiotics, 32:642-652 (1980)], influenced neither the tumor yield nor the polymorphonuclear leukocyte count. Baba et al., supra.

There are numerous reports on the antitumor effect of sulfated polysaccharides, including heparin [Jolles et al., Acta Univ. Int. Cancer, 16:682-685 (1960); Suemasu et al., Gann. 61(2):125-130 (1970)], sulfated laminaran and dextran [Jolles et al., British Journal of Cancer, 17:109-115 (1963)]. Yamamoto et al., in Japanese Journal of Experimental Medicine, 54:143-151 (1984), reported enhancement of antitumor activity of a fucoidan fraction by further sulfation. The sulfated product demonstrated activity against L-1210 leukemia. Polysaccharides with sulfate groups are also reported to be human T cell mitogens and murine polyclonal B cell activators. Sugawara et al., Microbiological Immunology, 28(7):831-839 (1984). Generally, homo-polysaccharides of high molecular weight with sulfate groups possess these properties. Dorries, European Journal of Immunology, 4:230-233 (1974); Sugawara et al., Cell Immunology, 74:162-171 (1982).

It has been reported that glucan extracted from the yeast Saccharomyces cervisiae is a modulator of cellular and humoral immunity. Wooles et al., Science 142:1078-1080 (1963). The polysaccharide also stimulated proliferation of murine pluripotent hematopoietic stem cells, granulocyte macrophage colony-forming cells and cells forming myeloid and erythroid colonies. Pospisil et al., Experientia, 38:1232-1234 (1982); Burgaleta, Cancer Research, 37:1739-1742 (1977). Maisin et al., [Radiation Research, 105:276-281 (1986)] also reported that IV administration of a polysaccharide induced protection of murine hematopoietic stem cells against x-ray exposure, thereby decreasing the mortality of the mice so exposed.

Lackovic et al., [Proceedings of the Society for Experimental Biology and Medicine, 134:874-879 (1970)], took yeast cell-wall and extracted all constituent matter leaving only "mannans" that he found to be responsible for the induction of α-interferon production by monocytes. The "purified mannans" alleged to be responsible for the physiologic response had a molecular weight of 5,500-20,000 daltons.

Seljelid et al., [Experimental Cell Research, 131(1):121-129 (1981)] have observed that insoluble or gel-forming glycans activated macrophages in vitro, whereas the corresponding soluble glycans did not. Bogwald, [Scandinavian Journal of Immunology, 20:355-360 (1984)] immobilized glycans that had a stimulatory effect on the macrophages in vitro. This led the authors to believe that the spatial arrangement of the glycan was decisive for the effect on the macrophages in vitro. A purified polysaccharide isolated from Candida albicans induced an antibody response by human peripheral blood lymphocytes in vitro. Wirz et al., Clinical Immunology and Immunopathology, 33:199-209 (1984). There were significant differences between the anti-Candida antibodies in sera of normal and Candida-infected individuals. Wirz et al., supra.

The antiviral activity of polysaccharides and polysaccharides linked to peptides has been observed. Suzuki et al., Journal of Antibiotics, 32:1336-1345 (1979). Suzuki et al., supra, reported an antiviral action of peptidomannan (KS-2) extracted from mycelial culture of Lentinus edodes. Both oral and intraperitoneal administration increased the peak serum interferon titer, which protected mice against viral infections. This was different from dextran phosphate (DP-40) [Suzuki et al., Proceedings of the Society for Experimental Biology and Medicine, 149(4):1069-1075 (1975)] and 9-methylstreptimidone (9-MS) [Saito et al., Antimier. Agent & Chemotherapy, 10(1):14-19 (1976)], which induced higher titers of interferon in mice only if administered IV or IP.

Anti-inflammatory activity of Aloe vera gel has been widely reported by both oral testimonies and respected scientific journals. Rubel [Cosmetics and Toiletries, 98:109-114 (1983)] discussed fully the possible mechanism of the anti-inflammatory effect of aloe gel. Ukai et al., [Journal of Pharmacobio-Dynamics, 6(12):983-990 (1983)] noted anti-inflammatory activity of polysaccharides extracted from the fruiting bodies of several fungi. The polysaccharides demonstrated a significant inhibitory effect on carrageenan-induced edema. One of the polymers, O-acetylated-D-mannan (T-2-HN), in addition demonstrated a more marked inhibitory effect than phenylbutazone on scald hyperalgesia. Ukai et al., supra.

Other researchers have also reported anti-inflammatory effects of complex polysaccharides [Saeki et al., Japanese Journal of Pharmacology, 24(1):109-118 (1974)], glycoproteins [Arita et al., Journal of Biochemistry, 76(4):861-869 (1974)] and sulfated polysaccharides [Rocha et al., Biochemical Pharmacology, 18:1285-1295 (1969)].

The controversy over whether the polysaccharide is a glucomannan, mannan, pectin, or of some other composition, is resolved by a series of chemical purification steps. Yagi et al., [Planta Medica, 31(1):17-20 (1977)], using a slightly modified extraction method, isolated acetylated mannan (aloe mannan) from Aloe arborescens Miller var. natalensis. Ovodova [Khim. Prior. Soedin, 11(1):325-331 (1975)], however, earlier isolated pectin as the main component of the same aloe species.

The structure of these immunologically active polysaccharides and the types of structural variations appear to be the factors that control their potency and toxicity. Their mode(s) of action remain poorly understood; however, recent evidence indicates that several polysaccharides induce lymphocytes and macrophages to produce a wide range of immunologically active substances. For example, 2-keto-3-deoxy-D-manno-octulosonic acid (KDO) appears to be the chemical portion of lipopolysaccharide (LPS) that provides the minimum signal for macrophage host defense activation [Lebbar et al., Eur. J. Immunol. 16(1):87-91 (1986)]. The composition of the present invention possesses all of the attributes of these immunologically active substances; it is among the most potent of all known biologically active polysaccharides but differs in that no toxicity has been observed. It also manifests specific antiviral activity through alteration of viral glycoprotein synthesis.

A number of pharmacology studies have been conducted on Aloe vera gel in recent times. Results have included more rapid healing of radiation burns [Rowe, J. Am. Pharm. Assoc., 29:348-350 (1940)] and accelerated healing of wounds [Lushbaugh et al., Cancer, 6:690-698 (1953)]. Thermal burns treated with Aloe vera gel heal much faster than untreated burns [Ashley et al., Plast. Reconstr. Surg., 20:383-396 (1957), Rovatto, supra, Rodriguez-Bigas et al., J.Plast. Reconstr. Surg., 81:386-389 (1988)]. The gel is useful in treating leg ulcers [E1 Zawahry et al., Int. J. Dermatol., 12:68-73 (1973)] and in hastening post surgical healing (Payne, Thesis submitted to Faculty of Baylor University, Waco, TX, MS Degree). Experimental evidence suggests that extracts of Aloe vera have anti-infectious properties [Solar, Arch. Inst. Pasteur Madagascar, 47:9-39 (1979)] and enhance phagocytosis [Stepanova, Fiziol. Akt. Veshchestva, 9:94-97 (1977)].

The active fraction of Aloe vera gel has been identified by Carrington Laboratories, Inc., Irving, Texas, as a long-chain polydispersed β-(1,4)-linked acetylated mannan intersperse with O-acetyl groups having a mannose monomer-to-acetyl group ratio of approximately 1:0.91. Acemannan is the nonproprietary name of the biologically active component of Carrisyn^{®}, a compound isolated and developed by Carrington Laboratories, Inc. See U.S. Patent No. 4,735,935, U.S. Patent No. 4,851,224, and the U.S. Patent Application Serial No. 07/229,164, and references cited therein, the disclosures of all of which are incorporated herein by reference.

Mannans, including glucomannans and galactomannans, have long been used by man. For example, galactomannans, in the form of plant gums, are widely employed as binders for control of food texture. In addition, some mannans have exhibited significant therapeutic properties (Davis and Lewis, eds. Jeanes A., Hodge J., In: American Chemical Society Symposium, Series 15. Washington, DC, American Chemical Society, 1975).

Pure mannans are relatively uncommon in higher plants, although they are a major structural component of some yeasts. For example, about 45% of the cell wall of Saccharomyces cerevisiae consists of a mannan. This mannan is a water soluble molecule composed of β-(1,6)-, β-(1,3)-, and β-(1,2)-linked, partially phosphorylated D-mannose residues [McMurrough et al., Biochem. J., 105:189-203 (1967)]. Other biologically active mannans have been obtained from Candida utilis [Oka et al., Gann, 60:287-293 (1969), Oka et al., Gann, 58:35-42 (1968)], Candida albicans, Coccidioides immitis and Rhodotorulum rubrum [Wheat et al., Infect. Immun., 41:728-734, (1983)]. Mannans (including galactomannans and glucomannans) are relatively resistant to attack by mannosidases but can be degraded by exo- and endo-mannanases [Emi, et al., Agr. Biol. Chem., 36:991-1001 (1972), Snaith, et al., Adv. Carbohydr. Chem. Biochem., 28:401-445, (1973) Herman, Am. J. Clin. Nutr., 24:488-498 (1971), McMaster, et al., Proc. Soc. Exp. Biol. Med., 135:87-90 (1970), Jones et al., J. Biol. Chem., 243:2442-2446 (1968), Eriksson et al., Acta. Chem. Scand., 22:1924-1934 (1968)]. The most marked biological activities of mannans in mammals are activation of macrophages and stimulation of T cells. As a result, they are potent immunostimulants with significant activity against infectious diseases and tumors [Hasenclever et al., J. Immun., 93:763-771 (1964)].

Saccharomyces mannan (15 mg/kg/day) enhances carbon clearance in normal male ddI mice, presumably acting as a reticuloendothelial system stimulant [Suzuki et al., Gann, 62:553-556 (1971)]. This same mannan also increases the number of antibody-forming cells in the spleen [Suzuki et al., Gann, 62:343-352 (1971)]. In vitro studies with mouse peritoneal cells (a mixture of macrophages and lymphocytes) indicate that some mannans and mannan-protein complexes can stimulate interferon release both in vitro and in vitro [Lackovic et al., Proc. Soc. Exp. Biol. Med., 134:874-879 (1970)]. The mannans stimulated interferon release in a manner similar to endotoxins but, in contrast to endotoxins, caused minimal toxicity (Borecky et al., Acta Virol., 11:264-266 (1967), Hasenclever, supra). The mannan from Candida albicans is active in this way, but the mannan from Saccharomyces cerevisiae is inactive [DeClercq et al., Ann. NY Acad. Sci., 173:444-461 (1970)]. Inconsistent or poor results have been obtained in other laboratories (DeClercq, supra). These differences may be due to slight structural or size differences in the polymers [Suzuki et al., Jpn. J. Microbiol., 12:19-24 (1968)]. The latter is more likely responsible since low molecular weight mannans (5.5-20 kDa) tend to be most active in the interferon-inducing assay, also Saccharomyces mannan tends to be larger than Candida mannan.

A galactomannan of 20 kDa from Lipomyces starkeyi had weak interferon-inducing properties. In contrast, Candida albicans mannan induced the appearance of interferon activity 2-24 hrs after intravenous administration (Borecky, supra).

DMG, a degraded mannoglucan from Microellobosporia grisea culture fluid, can stimulate cytotoxic activities of macrophages, natural killer (NK) cells and killer T cells, and it enhances the secretion of interleukin-1 (IL-1) and colony-stimulating factors (CSF). It has more potent antitumor activity than lentinan (a glucan from Lentinus edodes) [Nakajima et al., Gann, 75:260-268, (1984), Inoue et al., Carbohyd. Res., 114:164-168 (1983)]. DMG stimulates macrophages to produce increased amounts of IL-1. In addition, DMG enhances 1) antibody production against sheep erythrocytes, 2) natural killer activity of spleen as well as of peritoneal cells, and 3) cytostatic activity of peritoneal macrophages [Nakajima et al., Gann, 75:253-259 (1984)].

In humans, the major mannose-binding protein is an acute-phase protein; its levels rise in stressed individuals [Ezekowitz et al., J. Exp. Med., 169:185-196 (1989)]. The envelope glycoproteins of the human immunodeficiency virus (HIV gp120 and gp41) contain mannose-rich oligosaccharides that appear to be potential ligands for the mannose-binding protein. As a result, the mannose-binding protein can inhibit HIV infection of lymphoblasts and bind selectively to HIV-infected cells. Free yeast mannan can competitively interfere with binding of this protein to infected cells. Thus, factors that induce an increase in the level of the mannose-binding protein may confer protection against HIV.

### DETAILED DESCRIPTION OF PREFERRED EMBODIMENTS

Carrisyn^{®} is the brand name given by the assignee of the instant invention to the purified ethyl alcohol extract of the inner gel of the leaves of Aloe barbadensis Miller. The active component of Carrisyn^{®} has been designated "acemannan" by the United States Adopted Name Council. Not less than 73% of Carrisyn^{®} extract is acemannan; Carrisyn^{®} extract comprises generally about 73% to 90% acemannan. Carrisyn^{®} extract is generally produced by removing the outer sheath of the leaf, then removing and processing the inner filet or mucilage as follows: pH adjustment, ethanol extraction, freeze drying and grinding. See U.S. Application Serial No. 144,872 filed January 1988, a continuation-in-part of U.S. Application Serial No. 869,261 (now U.S. Patent No. 4,735,935), the disclosures of all of which are incorporated herein by reference. Processing in this manner predicts that essentially no covalent bonds are altered and therefore no toxic compounds are created. These manufacturing steps were developed to overcome the inability of traditional aloe product producers to standardize and stabilize the polysaccharides.

Carrisyn is a fluffy, white, amorphous powder, which is poorly soluble in water and dimethyl sulfoxide and insoluble in most other organic solvents. This powder contains not less than 73% of a polysaccharide consisting essentially of linear β(1-4)-D-mannosyl units. The polysaccharide is a long chain polymer interspersed randomly with acetyl groups linked to the polymer through an oxygen atom. The generic name for the polymer is acemannan. The degree of acetylation is approximately 0.91 acetyl groups per monomer as determined by the alkaline hydroxamate method. See Hestrin, Journal of Biological Chemistry, 180:240-261 (1949). Neutral sugars linkage analysis indicates that attached to the chain, probably through an α (1-6) linkage, is a D-galactopyranose in the ratio of approximately one for every 70 sugars. The 20:1 ratio of mannose to galactose indicates that galactose units are also linked together, primarily by a β(1-4) glycosidic bond. The chemical structure of acemannan may be represented as follows:

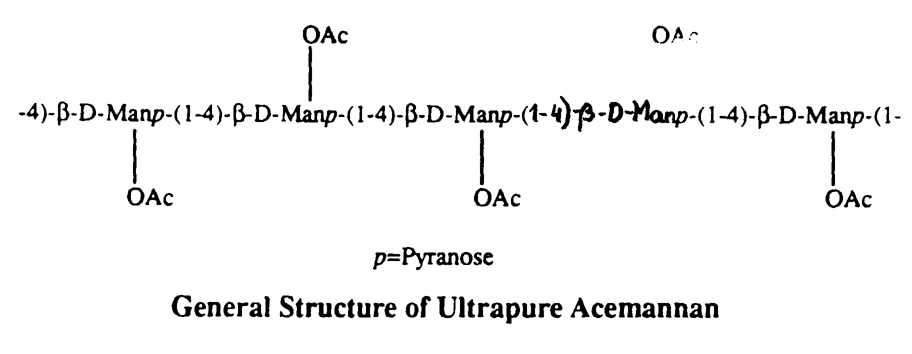

### DEFINITION OF TERMS

The term glycosylation" means the addition of carbohydrate molecules to a protein molecule. An acetylated mannan derivative, in particular acemannan, may exert its therapeutic effect by two possible mechanisms. One is the altering of glycosylation, such as inhibition of glucosidase I or the incorporation of the acetylated mannan derivative into glycoprotein.

The administration of acetylated mannan derivative can be achieved by topical application, oral ingestion, IP route, IV route or other parenteral routes of administration.

Not only can the acetylated mannan derivative be given to the recipient as a single agent, it can also be used in combination with other known therapeutic agents that are characterized by their requirement of the participation or aid of the host's immune system to achieve their maximal therapeutic effect.

Acemannan has now been discovered to be a potent inducer of IL-I and prostaglandin E2 (PGE2) production by human peripheral blood adherent cells in culture. The instant invention is believed to be the first practical non-toxic stimulator of IL-I release. IL-I is an important macrophage product reported in the literature to influence the activity and production of lymphocytes, fibroblasts , B-lymphocytes and endothelial cells. See Old,. Scientific American, 258(5):59-60, 69-75 (1988).

IL-I induces fibroblast proliferation which is fundamental to wound healing. IL-I also: (1) enhances bone marrow activity; it may be therapeutic in individuals whose bone-marrow is depressed; and (2) enhances the immune system in general.

A series of experiments with mixed lymphocyte cultures (MLC) has shown that acemannan increases the alloantigenic response of these lymphocytes in a dose-related fashion. Incubation of acemannan with monocytes permitted monocyte-driven signals to enhance the T lymphocyte response to lectin. Related studies on acemannan's effects on MLC have shown an increase in phagocytosis and activity of natural killer cells. Thus, in these in vitro test systems, acemannan is non-toxic and is an immunoenhancer.

Acemannan actively stimulates lymphocytes to secrete lymphokines and also causes HIV-infected lymphocytes to produce altered glycoproteins (GP-120) by a mechanism similar to that of glucosidase I inhibitors. See Gruters et al., Nature 330:74-77 (1987) and Pal et al., Intervirol. 30:27-35 (1989). Acemannan is phagocytized and apparently pumped to the Golgi/glycoprotein apparatus of the monocyte where it interferes directly with glycoprotein synthesis.

### A. Toxicology

The toxicological effects of acemannan have been studied in both in vitro and in vitro systems. Acemannan is not mutagenic or blastogenic in in vitro test systems. In vitro, the compound was non-toxic for H-9, MT-2 and CEM-SS lymphoid cells. In vitro toxicology studies on acemannan include a 91-day subchronic oral toxicity study in dogs, a 180-day chronic oral toxicity study in rats and an 180-day chronic oral toxicity study in humans. In these studies, no toxic effects were noted in dogs receiving up to 825 mg/kg of acemannan per day for 91 days. No clinical, gross pathologic or toxic effects were noted in rats receiving up to 38,475 ppm acemannan in their feed for 180 days. No adverse clinical or toxic effects were noted in human patients receiving 800 mg per day of acemannan for 180 days.

In pilot studies, administration of acemannan to dogs caused an absolute monocytosis in blood samples taken for complete white blood cell counts and morphology differential. Within 2 hours after oral administration of high doses of acemannan, large activated monocytes appeared in circulation. A similar effect has been observed in humans.

A study was performed using human peripheral blood monocyte cell cultures and ¹⁴C-labeled acemannan to track the incorporation or absorption of acemannan into a biological system. In this study, detectable amounts of ¹⁴C-labeled acemannan were absorbed or ingested by human peripheral monocyte/macrophage cells. Peak incorporation occurred at 48 hours. At a concentration of 5 mg/ml, the ¹⁴C-labeled acemannan was not cytotoxic to the monocyte/macrophage cells, and the weight/volume (w/v) digested cell mass was 760 times greater than the w/v of the digested acemannan solution. These results suggest that the macrophage is capable of maintaining intracellular concentration of acemannan at very high levels that are not cytotoxic.

A pyrogen assay was performed in rabbits in accordance with the pyrogen test protocol outlined in the U.S.P. XXI, Biological Test [151], using a 1 mg/ml injectable solution of acemannan. More frequent temperature measurements were taken than specified in the U.S.P. because of the unknown systemic effects of injected acemannan. Temperature changes in test animals did not exceed minimum changes allowed by the U.S.P. protocol; therefore, the solution met the U.S.P. requirements for absence of pyrogens. Acemannan injectable elicited a maximum body temperature increase of 0.3°C in one rabbit. This temperature rise occurred 90 minutes after injection. Acemannan is an inducer of IL-1 secretion by macrophages and monocytes in vitro. Since IL-1 is a potent pyrogen, this might explain the minimal, delayed temperature rise in this rabbit.

Twenty-four human subjects enrolled in and completed the study of the safety and tolerance of orally-administered acemannan. Clinical laboratory results showed that shifts out of the normal range occurred in the following: CO₂ in seven subjects, cholesterol in three subjects, triglycerides in two subjects, phosphorous in one, hemoglobin in four, basophils in two, monocytes in three, eosinophils in three, lymphocytes in four, neutrophils in two, and one each in red and white blood cells. Small numbers of red and white blood cells were also found in the urine. None of these shifts was clinically relevant.

Immune profile results showed group differences between Day 1 to Day 7 values for the following: CD-16, CD-4 (T-4), CD-8+Leu7, CD-4+CD-25, CD-8+CD-16, Leu7 and TQ-1. Mitogen responses were in the low range.

Vital signs did not appear to exceed normal ranges. There were no group differences in urine output. One subject in Group IV had diarrhea and loose stools during the study. One subject in Group I had loose stools during days 2 to 4 of the study. A total of 5 subjects reported a total of eight adverse events. All the events occurred in subjects receiving 1600 or 3200 mg oral acemannan daily for 6 days.

### B. Mode of Administration

The physical properties of acemannan allow it to be formulated and incorporated into all pharmaceutical dosage forms known to those skilled in the art. The biopharmaceutical and toxicological properties of acemannan permit it to be used in tissues and organs of living organisms and to be administered over a wide range of doses.

Acemannan may be administered to an animal orally, parenterally, topically and locally, in a daily dosage of 0.001 mg/kg to 1000 mg/kg body weight per day.

Mixed with suitable auxiliaries, acemannan may be compressed or filled into solid dosage units such as pills, tablets and coated tablets, or it may be processed into capsules. These oral dose forms would be administered at a dosage of about 0.1 mg/kg to 1000 mg/kg of body weight per day.

By means of suitable liquid vehicles, acemannan can be injected in solutions, suspensions or emulsions. These products would be administered at a rate of 0.001 mg/kg to 1000 mg/kg of body weight per day. As an adjunctive component of a vaccine or other product, acemannan would be used at a rate of 0.001 to 1000 mg per unit dose of adjuvanted product.

Topical administration of acemannan can be in the form of a processed gel, cream, lotion, solution, ointment or powder. These formulations could contain up to 90% acemannan.

### Example 1

### PHARMACOKINETIC BEHAVIOR OF ACEMANNAN

To evaluate the pharmacokinetic behavior of acemannan, ¹⁴C-labelled material was given by IP and IV injection and PO administration. Based on the results of previous pilot work, an aqueous dose of 200 mg ¹⁴C-labelled acemannan/200 ml with specific activity of 17.4 cpm/µg was administered to female dogs (approximately 20 mg/kg). Blood, urine and feces samples were taken at appropriate intervals for 48 hours or longer. Organ and tissue samples were taken after sacrifice, and all samples were analyzed for radioactivity using scintillation spectrometry.

Acemannan's kinetic behavior was typical of that seen with most pharmacologic agents; however, its biologic half-life (t_{1/2}) was extraordinarily long. Significant absorption occurred by all three routes of administration. Maximum blood levels were achieved after IV injection followed by 1P and then PO. Blood levels, which were immediately maximal at 200 µg/ml after IV injection, declined with a t_{1/2} of 50-60 hours; plasma levels were approximately twice those of blood. By comparison, after IP injection blood levels peaked at 45 µg/ml at 24 hours and then declined at a rate similar to that seen with IV; in fact, blood levels were nearly 90% maximal after only 8 hours. With oral administration, blood levels were measurable after 3 hours and peaked at 4-5 µg/ml. Based on the relatively long half-life in blood, a therapeutic dosing interval of approximately 7 days would be justified, considering the time required for three half-lives.

Radiolabeled acemannan distributed mainly in liver and spleen following IP or IV injection. Liver, marrow, thymus, and lymph nodes were primary sites of distribution after oral dosing, a finding consistent with the immunologic sites of action for acemannan. Levels of radiolabeled compound in tissues sampled after 48-52 hours ranged from a low of approximately 1 µg/g brain to a high of 85 µg/g spleen after IV injection. Interestingly, levels in brain and spinal cord were higher (approximately 3 µg/g tissue) after oral, compared to parenteral, administration. This could be the result of the liver's partial breakdown of the polymer into smaller molecular weight fractions during the first pass, thus rendering it capable of penetrating the blood-brain barrier.

In summary, with respect to clinical pharmacokinetic considerations, the data indicate that ¹⁴C-labelled acemannan (1) reaches peak blood levels within 8 hours or less by all routes studied, (2) has a relatively long biologic half-life, which would allow therapeutic dosing intervals of several days, and (3) achieves measurable levels in all tissue systems evaluated, including the central nervous system.

These pharmacokinetic data indicate that acemannan levels in blood and/or tissue can duplicate those levels known after injection or oral administration to produce therapeutic antitumor or antiviral effects in vitro. For example mice implanted with virally-infected Norman Murine Myxosarcoma (NMM) cells and injected IP within 24 hours with 1 mg/kg of acemannan showed 35% survival after 60 days compared to 0% survival in NMM-treated control mice (Peng et al., submitted for publication, 1990). Expected peak blood levels at an IP dose of 1 mg/kg would be on the order of 2 µg/ml (45 µg/ml x 1/20 mg/kg). Acemannan added to cultures of T-lymphocytes at a concentration of only 0.15 µg/ml (2.6¹⁰⁻⁹ M; 60,000 MW) increased the generation of cytotoxic T-cells 230% and increased the functional capacity of generated cytotoxic T-cells by 138% to destroy target cells against which they had been sensitized [Womble et al., Int. J. Immunopharmac. 10(8):967-974 (1988)]. Cytotoxic T-cells are thought to be generated against tumor cells like NMM cells.

Blood levels of 4-5 µg/ml obtained after oral administration of acemannan are also significant, since they correspond to the concentration of acemannan that gives optimal synergism with Zidovudine^{®} (AZT) in vitro. For example, alone 0.001 µg/ml AZT or 3.2 µg/ml acemannan increased the viability of CEM cells infected with HTLV-III_{RFII} virus by no more than 10%. Together the protective effect of the antiviral combination exceeded 70%. Similarly, a combination of 0.1 µg/ml of AZT and 1 mg/ml acemannan resulted in a protective effect exceeding 80% (Kemp et al. submitted for publication1990).

Thus, in conclusion, this pharmacokinetic study demonstrates that acemannan concentrations at least as great as those known to work in vitro are attainable in vivo.

### Example 2

### ACEMANNAN EFFECT OH CHOLESTEROL LEVELS IN ANIMALS AND HUMANS

Male dogs given 855 mg/kg/day of acemannan had a statistically significant reduction in serum cholesterol levels which was observed during a 91-day toxicity study. A similar effect was noted in normal male volunteers given doses of acemannan ranging from 400 mg to 3200 mg per day. An important effect of acemannan therapy which was observed was the statistically significant reduction in serum cholesterol level toward normal values for these subjects. Upon entry into the study, the mean cholesterol concentration of the 24 subjects was 189 mg/dl; it was 174 mg/dl upon exit. Statistical analysis using the "CRUNCH" Software version of the Wilcoxon Signed-Ranks Tests indicated >98% probability that the drop in cholesterol was not due to chance variation. Cholesterol concentrations in seven of the 24 patients decreased more than 20 mg/dl in 6 days of treatment, and therefore it is difficult to attribute this effect to a simple dietary improvement during the subjects' controlled residence during the study.

### SUMMARY

It is believed that the operation and administration of the present invention will be apparent from the foregoing description. While the method and techniques shown and described have been characterized as being preferred, it will be obvious that various changes and modifications may be made therein without departing from the scope of the invention as defined in the following claims.

## Claims

1. The use of an acetylated mannan in the manufacture of a medicament for regulating blood cholesterol levels in an animal.

2. The use of an acetylated mannan in the manufacture of a medicament for removing plaques formed in blood vessels of an animal.

3. The use of an acetylated mannan according to claim 1 or claim 2, wherein said acetylated mannan is acemannan.

## Patentansprüche

1. Verwendung eines acethylierten Mannans zur Herstellung eines Arzneimittels für die Regulierung des Blutcholesteringehalts im Tier.

2. Verwendung eines acethylierten Mannans zur Herstellung eines Arzneimittels für die Entfernung von in Blutgefäßen eines Tieres gebildeten Plaques.

3. Verwendung eines acethylierten Mannans nach Anspruch 1 oder 2, wobei das acethylierte Mannan Acemannan ist.

## Revendications

1. Utilisation d'un mannane acétylé pour la fabrication d'un médicament pour la régulation des niveaux de cholestérol sanguin chez un animal.

2. Utilisation d'un mannane acétylé pour la fabrication d'un médicament pour l'élimination des plaques formées dans les vaisseaux sanguins d'un animal.

3. Utilisation d'un mannane acétylé selon la revendication 1 ou la revendication 2, caractérisée en ce que le mannane acétylé est l'acémannane.
